(19) Europäisches Patentamt
European Patent Office
Office européen des brevets

(11) **EP 3 245 947 B1**

(12) **EUROPEAN PATENT SPECIFICATION**

(45) Date of publication and mention
of the grant of the patent:
**28.09.2022  Bulletin 2022/39**

(21) Application number: **17386020.6**

(22) Date of filing: **22.05.2017**

(51) International Patent Classification (IPC):
**A61B 5/02** (2006.01)      **A61B 5/053** (2021.01)

(52) Cooperative Patent Classification (CPC):
**A61B 5/02007; A61B 5/053**

(54) **NON-INVASIVE IMPEDANCE SPECTROSCOPY SYSTEM FOR EARLY DIAGNOSIS OF CORONARY ARTERY DISEASE**

NICHTINVASIVES IMPEDANZSPEKTROSKOPIESYSTEM ZUR FRÜHDIAGNOSE VON KORONARARTERIENERKRANKUNG

SYSTÈME DE SPECTROSCOPIE D'IMPÉDANCE NON INVASIF DE DIAGNOSTIC PRÉCOCE D'UNE MALADIE CORONARIENNE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.05.2016  GR 20160100261**

(43) Date of publication of application:
**22.11.2017  Bulletin 2017/47**

(73) Proprietor: **ARISTOTLE UNIVERSITY OF THESSALONIKI - E.L.K.E.,**
**Research Committee**
**54 636 Thessaloniki (GR)**

(72) Inventors:
• **Karapantsios, Theodoros**
  **GR-57013 Oreokastro Thessalonikis (GR)**
• **Evgenidis, Sotiris**
  **GR-57010 Chortiatis Thessalonikis (GR)**
• **Zacharias, Konstantinos**
  **GR-55337 Thessaloniki (GR)**
• **Karagiannis, Georgios**
  **GR-56728 Neapoli Thessalonikis (GR)**

(74) Representative: **Murgitroyd & Company**
**Murgitroyd House**
**165-169 Scotland Street**
**Glasgow G5 8PL (GB)**

(56) References cited:
**EP-A1- 3 005 942**        **WO-A1-2012/110042**
**US-A1- 2011 190 652**

• **Anonymous: "Impedance Spectroscopy device for early indication and diagnosis of Coronary disease (Cor---IS)", , 5 March 2015 (2015-03-05), pages 1-3, XP055357914, Retrieved from the Internet: URL:http://www.esa-tec.eu/workspace/assets /files/tdo0150-impedance-spectroscopy-54ff 3839edd0a.pdf [retrieved on 2017-03-23]**
• **Anonymous: "esa Technology Exchange", , 23 March 2017 (2017-03-23), pages 1-1, XP055357877, Retrieved from the Internet: URL:http://www.esa-tec.eu/see-more/technol ogies/?keywords=spectroscopy&category= [retrieved on 2017-03-23]**

**Description**

**Field of the invention**

The present invention concerns an innovative Impedance Spectroscopy system of high sensitivity and precision for early detection and diagnosis of coronary artery disease (CAD). The proposed system allows prompt and early evaluation of the functionality of the brachial or radial artery endothelium and consequently of the coronary artery network, therefore providing the diagnosis of coronary artery disease.

**Background of the invention**

Vascular endothelium is a single layer of flatten epithelial cells that cover the inner surface of the vascular wall. Until now we know that endothelium is not just a passive barrier between the blood and the vascular wall but a significant endocrine organ that plays a key role in the homeostasis of the cardiovascular system. In particular, endothelial cells secrete a vast number of mediator molecules that regulate the balance of: 1) vascular tone and the diameter of the vessel lumen (vasoconstriction / vasodilatation), 2) avoidance of thrombogenesis (antithrombotic / prothrombotic agents) and 3) inflammatory reaction of the vessels.

It has been clinically proved that when cardiovascular risk factors exist (diabetes, hypertension, dyslipidemia, smoking, obesity and severe family history) the normal endothelial function is disrupted and the vasoconstrictive, prothrombotic and inflammatory activity of endothelium prevails. This condition is called endothelial dysfunction and assists in mobilizing and maintaining the mechanisms of atherogenesis, thus increasing the long-term risk of future cardiovascular events. It is known that endothelial dysfunction plays a key role in all stages of atherogenesis, from the start-up of atherosclerotic process, even to the rupture of atherosclerotic plaque and in the case of the coronary network this translates in acute myocardial infarction.

Thus, the need was established to find a method of estimating and quantifying endothelial function and it was found that endothelium-dependent vasodilation induced by the release of nitric oxide is a good and measurable parameter with invasive or non-invasive techniques. Nitrogen monoxide (NO) is produced in the endothelial cells by L-arginine in the presence of enzyme NO endothelial synthase. Then is diffused into the smooth muscle cells and by increasing the concentration of cGMP (cyclic guanosine monophosphate), leads to a decrease in the intracellular concentration of $Ca^{+2}$ ions and vasodilation. Initially, invasive techniques (during coronary angiography) were used to measure endothelium-dependent coronary vasodilation in the coronary network, before and after the injection of agents (acetylcholine) that induce NO release from the coronary vascular endothelium. Coronary vessels with atherosclerotic lesions have been shown to exhibit endothelial dysfunction (decreased vasodilation or paradoxical vasoconstriction). The same was found in the coronary network of humans without known coronary artery disease but with severe risk factors. Also, it has been found that endothelial dysfunction is not limited to the coronary arteries, but covers the whole vascular system and further has been found that there's a strong correlation between endothelial function of coronary arteries with peripheral arteries (e.g. brachial, radial artery). As a result, this created the need to find means of assessment and quantification of endothelial function. It has been shown that the endothelial vasodilation induced by the release of nitric oxide is a good and measurable parameter with invasive or non-invasive techniques. Thus studies turned into the peripheral arterial system in order to examine the brachial artery initially by invasive methods (injection of endothelium active agents in brachial artery) and later by noninvasive methods such as FMD (Flow-Mediated Dilation). FMD is an ultrasound-based method, where the brachial arterial diameter is measured in response of endothelium-dependent vasodilatation caused by increased blood flow (Flow-Mediated Dilation) due to reactive hyperemia, after an increase in forearm shear stress (vascular occlusion) and vasoconstriction for a few minutes. The FMD value reflects the NO release from the endothelial cells and consequently the endothelial function.

However, the current FMD medical technique which is based on the ultrasound check of vasodilatation in the brachial artery has some vulnerabilities, that need to be dealt with in order the assessment of the endothelial function to become an everyday useful tool in the hands of clinical practitioners. Particularly, the FMD technique: 1) requires an expensive ultrasound device, 2) skilled personnel is needed for the measurements and is operator dependent, 3) has low image resolution compared to the vessel size, 4) has low repeatability and 4) exhibits poor imaging in obese people.

EP 3 005 942 A1 discloses an ultra-sensitive and accurate electrical impedance spectroscopy technique for the detection and characterization of bubbles in liquids that is required in numerous industrial processes as well as in the human's blood circulation during Decompression Sickness. In "Impedance Spectroscopy device for early indication and diagnosis of Coronary disease (Cor-IS)", 5 March 2015, pages 1-3, it is mentioned that a modified version of the system of EP 3 005 942 A1 may be used for the for early indication and diagnosis of Coronary disease. However, no further information was provided as to how this is to be achieved, nor on the type of modifications needed to be made to the system of EP 3 005 942 A1 to perform the desired function. WO2012110042A1 discusses a method and a system for carrying out the method of determining at least one cardiovascular quantity of a mammal.

The invention is set out in the appended set of claims. The present invention demonstrates high sensitivity for the detection of coronary artery disease and it can be used in risk assessment of cardiovascular events (unstable angina, myocardial infarction, ischemic stroke, sudden cardiac death), in patients with known coronary artery disease and with prognosis of risk restenosis after a percutaneous transluminal coronary angioplasty (PTCA) or coronary artery bypass graft (CABG). Moreover, since endothelial dysfunction is an early and potentially reversible disorder in the process of atherosclerosis, the present invention can be used in risk stratification in individuals without coronary artery disease but with atypical clinical symptomatology and health risk factors (elderly people, smokers, diabetes mellitus, arterial hypertension, dyslipidemia, obesity). Additional advantages of the method include the low cost of the device, simple application and availability even in remote areas distant from specialized medical centers. For the first time in clinical practice, the information provided by the impedance data time series allow the timely monitoring of the cardiovascular network's functionality. Therefore, the medical personnel not only can assess the endothelium functionality, but also can diagnose the severity of cardiovascular events. In addition, the current invention provides the ability to digitally monitor and record the patient's medical history, from the occurrence of the coronary artery disease and the progression during his/her life. Finally, it could be used to evaluate the response to specific therapeutic techniques (intensification of medication, modification of risk factors). Moreover, the present invention is simple to use and absolutely safe for humans. The application of digital signal processing techniques and custom developed software provides the ability that any signal analysis method can be applied and correlated with early prognosis or diagnosis of coronary heart disease. The latter is accomplished by simply upgrading the software code. The preliminary cost estimate shows that the current invention has a much lower cost than FMD ultrasound systems, making it accessible to the general public and capable of daily use. Additionally, the invention can be applied to various sites on the human body (e.g. thorax, carotid, etc.) providing independent or simultaneous examination. The use of ultrasound systems does not allow the simultaneous examination on different sites. Moreover, the present invention is substantially less bulky and lighter than ultrasound systems and as well as could be portable and battery-powered.

The invention is based on the non-invasive measurement of the electrical impedance of a medium, in particular the vessels surrounded by the human tissues and skin, by applying a high frequency alternating current (AC) having appropriate frequency, which is defined after frequency scanning (spectroscopy technique).

It can measure with unprecedented sensitivity not only the impedance variations caused by the ischemic cuff and release, but even the minuscule pulsatile impedance undulations of the blood flow. In the circulation network, during every heart systole a blood bolus volume on its passage leads to a temporal local vessel volume increase. The measured zone of a vessel of the circulatory network can be considered as an inner cylinder surrounded by tissues, which is filled with blood (inflow phase). Later during the diastole, the blood is transported further (outflow phase), but also returned via the venous network. In 1937 Sigman et al., were the first to report that the electrical resistivity and consequently the impedance of blood is flow dependent. They found that the resistivity fell about 7% when the blood velocity was increased from 10 to 40 cm/s.

But, no Sigman effect is found in plasma or electrolytes. The Sigman effect is due to the non-spherical bodies in the blood, in particular the erythrocytes. At higher velocities but still linear flow, the erythrocytes reorient into the flow direction, and in a tube (e.g. a vessel) they also clump together around the central axis. The erythrocyte orientation means less hindrance to electric current flow and lower resistivity if resistance is measured in the axial direction. These impedance changes are correlated with flow pulsation frequency (cardiac component) and that magnitude is becoming small above 10 Hz. In general and depending on measurement sites on the human body, the impedance time series can contain three major physiological components that provide useful information for diagnostics:

- a low frequency (LPF) respiratory component (fundamental < 0,5 Hz), dominant if measured at the chest,
- a band limited frequency (BPF) cardiac component (fundamental ~ 1-3 Hz),
- a high frequency component >10 Hz (HPF), harmonics of cardiac component and measurement noise,
- a calculated mean impedance (0 Hz, "DC component").

The higher frequency cardiac component is superimposed on the low frequency respiratory component. The higher frequency cardiac component represents the impedance change during each cardiac cycle that occurs immediately following the QRS deflection on the electrocardiogram. The low frequency respiratory component represents the impedance change during respiration, due to the expansion of the lungs and thorax. Moreover, each component has a different fundamental frequency, typically 1-3 Hz for cardiac activity and 0,1-1,0 Hz for respiratory activity. This frequency differentiation allows separation of each signal by specific filtering techniques (LPF, BPF and HPF time series signals). Mean impedance, is represented by a "DC shift" in impedance and changes according to the amount of static conductive fluid as a function of time (hours/days). More specifically, a change in impedance waveform morphologies may be an indicator of change in blood volume, interstitial volume or tissue integrity. Deviations in the impedance waveform morphologies such as in the positive or negative slope, time duration between minimum and maximum magnitudes, difference between the minimum and maximum magnitudes, changes in the minimum and maximum of first derivative, changes in the area under a specific waveform or other deviations in the waveform morphology of complex impedance may be indicative of a vector (complex impedance) specific change in chamber or vessel blood volume. The previous deviations could be present, in cases such as in heart disease, tissue degradation such as in myocardial ischemia, or interstitial fluid accumulation such as in peripheral or pulmonary edema and all secondary to cardiac, vascular or renal disease. Therefore, by measuring those impedance characteristics at various frequencies (spectroscopy technique), prior to ischemic cuff (reference measurement) and during the endothelium-dependent vasodilatation caused by increased blood flow due to reactive hyperemia, the blood flow characteristics can be determined. Arteries that suffer from atherosclerosis have impaired vasodilation (endothelial dysfunction) and decreased blood flow. Thus, by comparing the impedance characteristics in hyperemia phase with the characteristics prior to ischemic cuff, for the atherosclerotic arteries there is less variation than the healthy ones. Consequently, the present invention achieves a prompt and early diagnosis of the endothelium functionality of the brachial or radial artery and consequently of the coronary network, due to their similarity.

The electrical impedance can be measured with electrodes of different geometries such as ring electrodes, flat surfaces or any other kind of electrode that is not intrusive and can be attached on the human skin. Proper electrode dimensions as well as interelectrode distance are key parameters, since they determine the measurement volume inside the body and spatial resolution. Therefore the electrodes are placed along the brachial or radial artery on the right or left forearm. As regards electrode selection for the application of this invention for the detection of coronary artery disease, the first choice would be standard electrocardiograph (ECG) pads or encephalograph (EEG) electrodes adhered onto the human skin, providing non-invasive measurements.

Description of the drawings

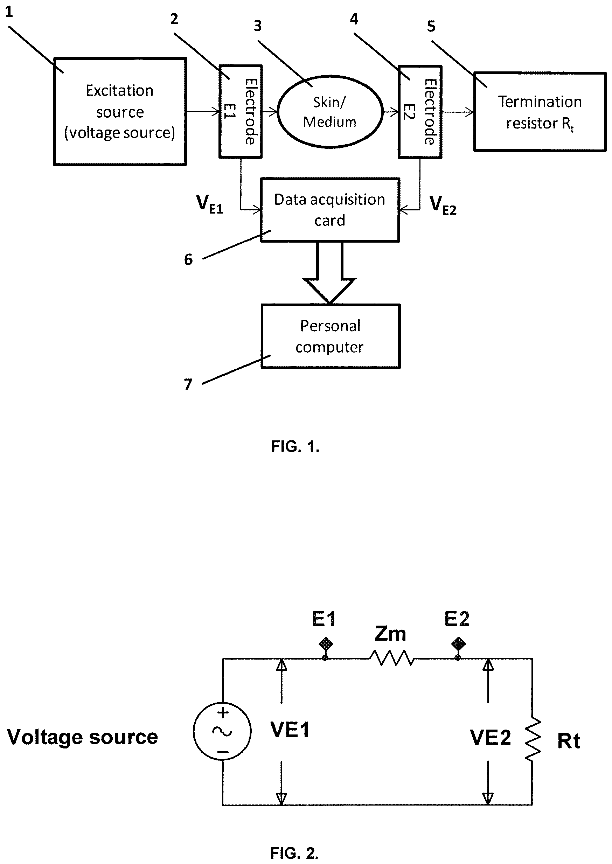

According to the invention, the applicable excitation mode via the electrodes uses a voltage source, the applied voltage is set at a constant value (voltage control). According to an example not covered by the claimed invention, the applicable excitation mode may use a current source, the applied current is set at a constant value (current control). Fig. 1 demonstrates the design logic (block diagram) for measuring the electrical impedance $Z_m$ or inversely the electrical admittance $Y_m$, of a medium composed of tissues, skin and forearm arteries, applying the voltage source excitation mode. Moreover, Fig. 2 shows the electrical equivalent of the measurement system presented in Fig. 1. $V_{E1}$ is the input voltage that excites electrically the medium is connected to the input electrode E1 and generates an input current passing through the skin/medium.

Regardless the excitation mode voltage or current, frequency scanning and preliminary testing can reveal the proper excitation frequency and amplitude that provide the highest measurement sensitivity and accuracy. Also, the hardware of the novel electrical impedance technique allows for employing different waveform types of excitation signal to the electrodes (continuous: sinusoidal, square, triangular, etc., or pulsed. The input current generated by the excitation voltage $V_{E1}$ creates a voltage drop on the impedance $Z_m$ of the medium, and exits from the output electrode E2. The output electrode is connected to the one end of the termination resistance $R_t$, while the other end of the resistance is connected to signal generator (common voltage point). If needed, the common point could be grounded. The voltage across the termination resistance is the measured output voltage $V_{E2}$. $Z_m$ and Rt constitute a voltage divider and the ratio k of the voltages is:

$$k = \frac{V_{E2}}{V_{E1}} \equiv \frac{R_t}{Z_m + R_t} \qquad (1)$$

Solving for $Z_m$, is obtained:

$$Z_m = \left(k^{-1} - 1\right) R_t \qquad (2)$$

Where $Z_m$ is the equivalent impedance as measured by the two electrodes E1 and E2.

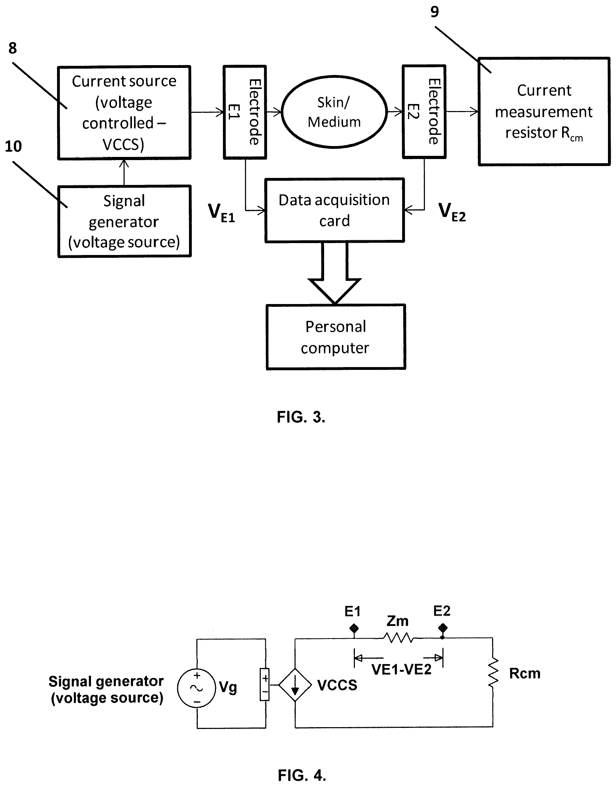

The current source excitation mode is accomplished by applying an alternating current source controlled by a voltage source (Voltage Controlled Current Source, VCCS). The VCCS is located between the voltage source and the electrode E1, as shown in Fig. 3. Fig. 4 shows the electrical equivalent of the measurement system shown in Fig. 3.

For the needs of the excitation, a proper AC current source (VCCS) of the desired frequency and amplitude is applied. $V_g$ is the output voltage of the signal generator, $V_m = V_{E1} - V_{E2}$ is the differential voltage measured across the electrodes E1 and E2 caused by the excitation current of the VCCS, $Z_m$ is the measured impedance between the two applied electrodes and $R_{cm}$ is an optional resistor for the measurement of excitation current. The VCCS is a component that converts any type of voltage source into a current source. The current is controlled by the voltage applied at the input. This feature is the great advantage of VCCS compared with a current source generator. Instead of using a current generator where the available waveform type would only be sinusoidal, the VCCS can convert any arbitrary waveform voltage. For example, continuous waves such as sinusoidal, square, triangular, etc., or pulse waves of any voltage amplitude and frequency can be converted. Although the vast majority of biomedical applications would require a sinusoidal waveform, this does not exclude the application of any other waveform type. Since the output of the VCCS is current and the input is voltage, therefore the transfer function, which is the output over the input, is in terms of $\Omega^{-1}$. These components are also called as transconductance devices and the most common use is as transconductance amplifiers (TA). The transconductance amplifier is like a typical voltage amplifier but the amplified output is a current. It

$$|H_{TA}| = \frac{I_o}{V_i} > 1$$

is an amplifier because the magnitude of the transfer function is , where $I_o$ is the output current and $V_i$ is the input voltage. For the application of the present invention on humans and because of safety reasons and regulations, the excitation current must not exceed 2 mA. Therefore, the majority of biomedical devices operate with a stimulation current having a typical value of 0,1 mA. As a result, the practical implementation of the VCCS would require the current output to be in mA range and the voltage input in V range. Hence the magnitude of the transfer function of VCCS $|H_{VCCS}|$ would be far less than one :

$$|H_{VCCS}| = \frac{I_{out}}{V_i} = \frac{mA}{V} \ll 1 \qquad (3)$$

As regards the application of present invention on humans, the magnitude of the transfer function or gain is set to be:

$$|H_{VCCS}| = \frac{1mA}{1V} = 0.001 \qquad (4)$$

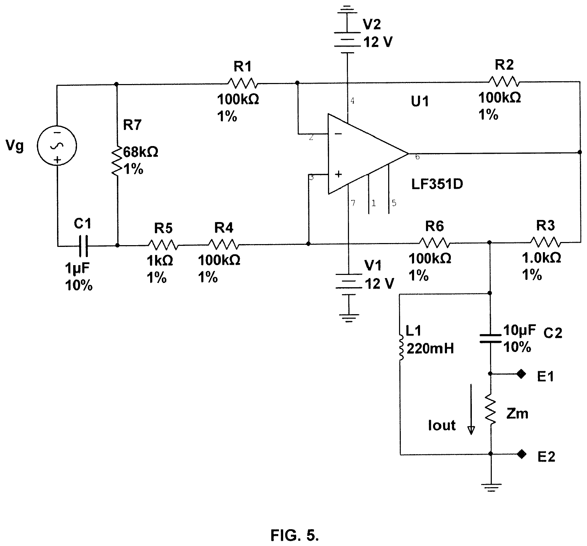

With that gain, 1 V of the voltage source is transformed (converted) into 1 mA current excitation passing through the electrode E1. In the case that it is considered necessary, the VCCS gain could be adjusted increased or decreased at any desired value. It is known from electrical circuits' theory that the current source has very high internal impedance (theoretically infinite) while the voltage source has very low internal impedance (theoretically zero). Therefore, the positive and the negative inputs of an operational amplifier could be used to constitute a current source. The VCCS is essentially a high impedance current source and many alternative designs of current sources and VCCS exist. Most of the designs are for current sources controlled by a DC voltage source. But for the demanding application of the endothelium functionality detection of the brachial or radial artery and consequently of the coronary artery network, where the practical problems involved are not so simple or/and obvious, a versatile and reliable alternating current source (AC current) is needed, with high output impedance and wide frequency range. In this invention, a novel, modified and improved alternating current VCCS circuit was designed and the schematic drawing is shown in Fig. 5. The gain is set by $R_3$ and ratio of $R_1/R_2$, which is typically 1/1. In the new improved design, the ratio of $(R_5+R_4)/(R_6+R_3)$ should be matched with $R_1/R_2$ for proper biasing of the operational amplifier, so:

$$\frac{R_5 + R_4}{R_6 + R_3} = \frac{R_1}{R_2} \qquad (5)$$

The gain then is:

$$|H_{VCCS}| = \frac{I_{out}}{V_g} = \frac{1}{R_3} \qquad (6)$$

Consequently, low values for R3 can be used and have all the other resistors high in value, such as 100 kΩ or 1 MΩ. Based on Equations 4 and 6, the value of the resistor would be $R_3=1$ kΩ. $R_3$ value may be decreased, if increased detection sensitivity is required. Therefore, the output impedance of the current source is very high, and even for typical operational amplifier with CMRR of 60dB, the output impedance would "degrade" to 10 MΩ, which is an acceptable value in biomedical applications. The majority of commercial operational amplifiers have CMRR (Common Mode Rejection Ratio) better than 80 dB, so the impedance is increased even more. This circuit can supply many milliamperes (or as low as microamperes) depending on the input voltage and VCCS gain. Also, with a power supply of at ± 12 V DC (nominal voltage values), the maximum resulting voltages caused by the output current, can be as large as 10 V, with good efficiency. If higher resulting voltages are needed, the power supply voltage can be increased.

Based on the electronic circuit design guidelines, from Equations 5 and 6 as well as on the gain requirement from Equation 4, the resistor values selected are those shown in Fig. 5. The operation amplifier is the integrated circuit LF351D, which is a JFET operational amplifier, with internal frequency compensation, high CMRR 100 dB, high slew rate 13 V/μs, low noise and distortion 0,003% (typical). Test measurements have shown that even for the case of a high value 1 kΩ load, the gain is dropped only about 0,4% and a phase shift of -5,7° is observed. But these deviations are negligible and do not affect the accuracy of the measurements because when applying the technique, a baseline measurement is always taken. Therefore, these changes are known and taken into account. The measured impedance as measured by the electrodes E1 and E2 (Fig. 4), is the magnitude of $Z_m$ and can be simply calculated as the ratio of the differential voltage (voltage drop) between E1-E2 and output current:

$$Z_m = \frac{V_{E1} - V_{E2}}{I_{out}} = \frac{V_m}{I_{out}} \qquad (7)$$

In the condition where there's no phase shift between the input and ouput current, then the impedance $Z_m$ is transformed to resistance $R_m$. The voltage difference $V_{E1}$-$V_{E2}$ is actually the measured key parameter ($I_{out}$ is constant), because it is directly affected by the impedance variation due to blood flow.

The AC current excitation is accomplished via the input electrode E1 while the voltage drop is measured between electrodes E1 and E2. The excitation and output voltages for the voltage source mode ($V_{E1}$ and $V_{E2}$), as well as the voltage drop for the current source mode ($V_{E1}$ - $V_{E2}$) are recorded by the data acquisition card having high sampling rate (192 kS/s) and resolution (24bit), providing two important advantages: a) measurement of electrical signals with maximum frequency in the order of several tens of kHz, with typical frequency range 1 - 96 kHz, due to the high sampling rate and b) measurement of extremely low voltages in the order of microvolts due to high resolution of 24 bit. None of the conventional electrical techniques combine the aforementioned advantages. With the present invention, the measurement sensitivity is improved several orders of magnitude, providing detection capability of very low impedance values of ~0,01%. Thus, it is possible to detect extremely small variations due to blood flow with unprecedented sensitivity.

The previously mentioned advantages of the invention are accomplished with the use of custom made software build for the purpose, which is an integral part and necessary for the operation of the device. The most important advantage of the electrical impedance spectroscopy technique concerns the digital processing of the acquired signals. This processing can take place either in real time or at a later time (post-processing). Therefore, the measured voltage drop is digitally filtered by applying a high order digital band pass filter centred at the excitation frequency with a nominal 3 kHz narrow bandwidth. This filtering suppresses any kind of electrical interference, resulting into high quality electrical signals for a wide range of impedance values and blood velocities. It should be emphasized that conventional electrical techniques either do not have filters for electrical interference rejection or these filters are not effective enough for reliable and accurate measurements. Next, because the accurate envelope detection of the measured voltage drop is a nonlinear process, the signal is digitally filtered by applying a low pass filter with a nominal cut-off frequency of 100 Hz, without any signal loss. It should be emphasized that conventional electrical techniques employing analogue electronics, either do not have filters for electrical interference rejection or these filters are not effective and selective enough, introducing in this way signal losses that affect both the accuracy and sensitivity of measurements. The particular feature of strong resistance to any form of electrical interference renders the present invention innovative and much more reliable than existing techniques.

The low pass filtered envelope of signals VE1 and VE2 is then used for the calculation of the impedance magnitude of $Z_m$ by applying Equations 2 or 7. Then the components of LPF, BPF and HPF are separated from the time series of $Z_m$ in the frequency domain with the use of appropriate filtering. Comparing the $Z_m$ values before and after the ischemic cuff allows the evaluation of the level of endothelium functionality. In any case, impedance time series carry crucial information in both frequency and time domain. Therefore, several statistical parameters and spectral features can be associated with endothelium-dependent vasodilatation caused by increased blood flow due to reactive hyperemia and finally the diagnosis of coronary artery disease. As a result, various methods of signal processing and statistical analysis can be applied to the resulted time series, e.g.: a) moments (mean, standard deviation and higher order moments), b) Fast Fourier Transform (FFT) spectral analysis algorithm, c) Spectrogram analysis, d) auto-correlation & cross-correlation and e) continuous and discrete wavelets transform (CWT & DWT). It should be pointed out that the previously mentioned signal analysis techniques are not exclusive. Any digital signal processing technique or method can be applied on the impedance time series in order to study the endothelium functionality and blood flow characteristics.

**Claims**

1. A system for early, non-invasive diagnosis of coronary artery disease of a patient with evaluation of endothelium functionality of brachial or radial artery and of coronary network, the system comprising:

   a device comprising:

   an excitation source (1) configured for generating an excitation input signal;
   a first and second non-invasive electrodes (2, 4) that are connected therewith to be attached on the skin (3) of said patient for generating signals, the electrodes comprising a first input electrode (2) and a second output electrode (4);
   a termination resistor (5) connected at one end to the second electrode and at a second end to a common voltage point such that the voltage across the termination resistor (5) is the measured output voltage;
   a data acquisition card (6); and

   a personal computer (7) implementing software for operation of the device and
   for digital processing, storage and transmission of acquired signal data;

   wherein the excitation source is configured to apply a high frequency alternating current of selected excitation frequency to excite electrically the skin connected to the first electrode and generate an input current passing through the skin of the patient, which exits from the second electrode;
   wherein the excitation frequency of the excitation source is variable between 1kHz and 96kHz to allow selection of an excitation frequency appropriate for measurement of variations of impedance in a medium, the medium being composed of tissue, blood vessels and skin, between the first and second electrodes, the electrodes being applied to a forearm of the patient along the brachial or radial artery;
   wherein the selected excitation frequency allows detection of variations in impedance in the medium due to blood flow;
   wherein the data acquisition card (6) is configured for recording signal data generated by the device over a sample time period, the signal data comprising an input voltage signal measured across the excitation source, and an output voltage signal measured across the termination resistor (5);
   wherein the personal computer is configured to:
   receive recorded signal data, the recorded signal data including data recorded over the sample time period, during application of the electrodes to the forearm of the patient along the brachial or radial artery, wherein upon execution of the software, the computer is further

   configured to:

   process the input and output voltages to determine impedance of the medium between the first and second electrodes over the sample time period, including variations in impedance, said processing including (i) digitally filtering the signals by applying a high order digital band pass filter centered at the excitation frequency and having a narrow bandwidth, to suppress any electrical interference and (ii) digitally filtering the signal by applying a low pass filter without signal loss;
   calculate the impedance time series;
   filter the impedance time series in the frequency domain to separate a set of physiological components comprising a low frequency, LPF, respiratory component, a band limited frequency component, BPF, and a high frequency component ,HPF;
   store, display and transmit results obtained by processing, including the impedance time series and impedance values before and after application of the ischemic cuff to allow evaluation of the endothelium functionality.

2. The system according to claim 1, **characterized in that** the excitation source consists of a voltage source (1) wherein the output electrode (4) is connected to one end of the termination resistor (5) and the other end of the resistor is connected to a signal generator.

3. The system according to any one of the preceding claims, **characterized in that** the resistance of the termination resistor (5) and the impedance value of the medium, namely the skin, tissue and blood vessels of the patient, between the first and second electrodes (2,4) constitute a voltage divider.

4. The system according to any one of the preceding claims, **characterized in that** the data acquisition card (6) has a high sampling frequency 192kS/s and a 24 bit resolution.


**Patentansprüche**

1. Ein System zur frühen, nicht-invasiven Diagnose koronarer Herzkrankheit eines Patienten mit Evaluierung von Endothelfunktionalität brachialer oder radialer Arterie und des Herzgeflechts, wobei das System Folgendes beinhaltet:

   eine Vorrichtung, die Folgendes beinhaltet:

   eine Anregungsquelle (1), die konfiguriert ist, um ein Anregungseingangssignal zu erzeugen;
   eine erste und eine zweite nicht-invasive Elektrode (2, 4), die damit verbunden sind, um an der Haut (3) des Patienten angeklebt zu werden, um Signale zu erzeugen, wobei die Elektroden eine erste Eingangselektrode (2) und eine zweite, Ausgangselektrode (4) beinhalten;
   einen Terminationswiderstand (5), der an einem Ende mit der zweiten Elektrode und an einem zweiten Ende mit einem gemeinsamen Spannungspunkt verbunden ist, sodass die Spannung über den Terminationswiderstand (5) hinweg die gemessene Ausgangsspannung ist;
   eine Datenerlangungskarte (6); und

   einen Personalcomputer (7), der Software zum Betrieb der Vorrichtung und zur digitalen Verarbeitung, Speicherung und Übertragung erlangter Signaldaten implementiert;
   wobei die Anregungsquelle konfiguriert ist, um einen Wechselstrom hoher Frequenz ausgewählter Anregungsfrequenz aufzubringen, um die Haut, die mit der ersten Elektrode verbunden ist, elektrisch anzuregen und einen Eingangsstrom zu erzeugen, der durch die Haut des Patienten geht, aus der zweiten Elektrode austretend;
   wobei die Anregungsfrequenz der Anregungsquelle zwischen 1 kHz und 96 kHz variabel ist, um eine Auswahl einer Anregungsfrequenz zu ermöglichen, die für die Messung von Variationen von Impedanz in einem Medium geeignet ist, wobei das Medium aus Gewebe, Blutgefäßen und Haut zwischen der ersten und zweiten Elektrode besteht, wobei die Elektroden auf einen Unterarm des Patienten entlang der brachialen oder radialen Arterie aufgebracht werden;
   wobei die ausgewählte Anregungsfrequenz die Detektion von Variationen der Impedanz in dem Medium aufgrund des Blutflusses ermöglicht;
   wobei die Datenerlangungskarte (6) konfiguriert ist, um Signaldaten, die durch die Vorrichtung über einen Probennahmezeitraum erzeugt werden, aufzuzeichnen, wobei die Signaldaten ein Eingangsspannungssignal, das über die Anregungsquelle hinweg gemessen wird, und ein Ausgangsspannungssignal, das über den Terminationswiderstand (5) hinweg gemessen wird, beinhalten;
   wobei der Personalcomputer für Folgendes konfiguriert ist:
   Empfangen aufgezeichneter Signaldaten, wobei die aufgezeichneten Signaldaten Daten umfassen, die über den Probennahmezeitraum während des Aufbringens der Elektroden auf den Unterarm des Patienten entlang der brachialen oder radialen Arterie aufgezeichnet werden,
   wobei bei der Ausführung der Software der Computer ferner für Folgendes konfiguriert ist:

   Verarbeiten der Eingangs- und Ausgangsspannung, um die Impedanz des Mediums zwischen der ersten und zweiten Elektrode über den Probennahmezeitraum einschließlich Variationen der Impedanz zu bestimmen, wobei das Verarbeiten Folgendes umfasst: (i) digitales Filtern der Signale durch das Aufbringen eines digitalen Bandpassfilters hoher Ordnung, der auf die Anregungsfrequenz zentriert ist und eine schmale Bandbreite aufweist, um jegliche elektrische Interferenz zu unterdrücken, und (ii) digitales Filtern des Signals durch das Aufbringen eines niedrigen Passfilters ohne Signalverlust;
   Berechnen der Impedanzzeitserie;
   Filtern der Impedanzzeitserie in dem Frequenzbereich, um einen Satz physiologischer Komponenten, der eine Komponente niedriger Atemfrequenz, LPF, eine bandbegrenzte Frequenzkomponente, BPF, und eine Komponente hoher Frequenz, HPF, beinhaltet, zu trennen;

Speichern, Anzeigen und Übertragen von Ergebnissen, die durch das Verarbeiten erhalten werden, einschließlich der Impedanzzeitserie und der Impedanzwerte vor und nach der Aufbringung der ischämischen Manschette, um die Evaluierung der Endothelfunktionalität zu ermöglichen.

**2.** System gemäß Anspruch 1, **dadurch gekennzeichnet, dass** die Anregungsquelle aus einer Spannungsquelle (1) besteht, wobei die Ausgangselektrode (4) mit einem Ende des Terminationswiderstands (5) verbunden ist und das andere Ende des Widerstands mit einem Signalerzeuger verbunden ist.

**3.** System gemäß einem der vorhergehenden Ansprüche, **dadurch gekennzeichnet, dass** der Widerstand des Terminatorwiderstands (5) und der Impedanzwert des Mediums, nämlich der Haut, des Gewebes und der Blutgefäße des Patienten, zwischen der ersten und zweiten Elektrode (2, 4) einen Spannungsteiler darstellen.

**4.** System gemäß einem der vorhergehenden Ansprüche, **dadurch gekennzeichnet, dass** die Datenerlangungskarte (6) eine hohe Abtastfrequenz 192 kS/s und eine Auflösung von 24 Bit aufweist.

**Revendications**

**1.** Un système pour le diagnostic précoce, non invasif, d'une maladie coronarienne d'un patient avec évaluation de la fonction endothéliale d'artère brachiale ou radiale et du réseau coronaire, le système comprenant :

un dispositif comprenant :

une source d'excitation (1) configurée pour la génération d'un signal d'entrée d'excitation ;
des première et deuxième électrodes non invasives (2, 4) qui sont connectées à celle-ci pour être fixées sur la peau (3) dudit patient pour la génération de signaux, les électrodes comprenant une première électrode d'entrée (2) et une deuxième électrode de sortie (4) ;
une résistance de terminaison (5) connectée au niveau d'une extrémité à la deuxième électrode et au niveau d'une deuxième extrémité à un point de tension commun de telle sorte que la tension de part et d'autre de la résistance de terminaison (5) est la tension de sortie mesurée ;
une carte d'acquisition de données (6) ; et

un ordinateur personnel (7) mettant en œuvre un logiciel pour le fonctionnement du dispositif et pour le traitement numérique, le stockage et la transmission de données de signaux acquises ;
dans lequel la source d'excitation est configurée pour appliquer un courant alternatif haute fréquence de fréquence d'excitation sélectionnée pour exciter électriquement la peau connectée à la première électrode et générer un courant d'entrée traversant la peau du patient, lequel sort par la deuxième électrode ;
dans lequel la fréquence d'excitation de la source d'excitation peut varier entre 1 kHz et 96 kHz pour permettre la sélection d'une fréquence d'excitation appropriée pour la mesure de variations d'impédance dans un milieu, le milieu étant composé de tissu, de vaisseaux sanguins et de peau, entre les première et deuxième électrodes, les électrodes étant appliquées sur un avant-bras du patient le long de l'artère brachiale ou radiale ;
dans lequel la fréquence d'excitation sélectionnée permet la détection de variations d'impédance dans le milieu du fait du débit sanguin ;
dans lequel la carte d'acquisition de données (6) est configurée pour l'enregistrement de données de signaux générées par le dispositif sur une plage de temps échantillon, les données de signaux comprenant un signal de tension d'entrée mesuré de part et d'autre de la source d'excitation, et un signal de tension de sortie mesuré de part et d'autre de la résistance de terminaison (5) ;
dans lequel l'ordinateur personnel est configuré pour :
recevoir des données de signaux enregistrées, les données de signaux enregistrées incluant des données enregistrées sur la plage de temps échantillon, durant l'application des électrodes sur l'avant-bras du patient le long de l'artère brachiale ou radiale,
dans lequel à l'exécution du logiciel, l'ordinateur est en outre configuré pour :

traiter les tensions d'entrée et de sortie pour déterminer l'impédance du milieu entre les première et deuxième électrodes sur la plage de temps échantillon, y compris des variations d'impédance, ledit traitement incluant (i) le filtrage numérique des signaux par application d'un filtre passe-bande numérique d'ordre élevé centré à la fréquence d'excitation et ayant une largeur de bande étroite,
pour supprimer toute interférence électrique et (ii) le filtrage numérique du signal par application d'un filtre passe-bas sans perte de signal ;
calculer la série temporelle d'impédance ;
filtrer la série temporelle d'impédance dans le domaine fréquentiel pour séparer un ensemble de composants physiologiques comprenant un composant respiratoire basse fréquence, LPF, un composant à fréquence à bande réduite, BPF, et un composant haute fréquence, HPF ;
stocker, afficher et transmettre les résultats obtenus par traitement, y compris la série temporelle d'impédance et les valeurs d'impédance avant et après l'application du ballonnet ischémique pour permettre l'évaluation de la fonction endothéliale.

2. Le système selon la revendication 1, **caractérisé en ce que** la source d'excitation consiste en une source de tension (1), l'électrode de sortie (4) étant connectée à une extrémité de la résistance de terminaison (5) et l'autre extrémité de la résistance étant connectée à un générateur de signal.

3. Le système selon l'une quelconque des revendications précédentes, **caractérisé en ce que** la résistance de la résistance de terminaison (5) et la valeur d'impédance du milieu, à savoir la peau, le tissu et les vaisseaux sanguins du patient, entre les première et deuxième électrodes (2, 4), constituent un diviseur de tension.

4. Le système selon l'une quelconque des revendications précédentes, **caractérisé en ce que** la carte d'acquisition de données (6) a une fréquence d'échantillonnage élevée de 192 kS/s et une résolution de 24 bits.

**FIG. 1.**

**FIG. 2.**

**FIG. 3.**

**FIG. 4.**

FIG. 5.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3005942 A1

- WO 2012110042 A1

**Non-patent literature cited in the description**

- Impedance Spectroscopy device for early indication and diagnosis of Coronary disease (Cor-IS). *Decompression Sickness,* 05 March 2015, 1-3